# FASCICULE DE BREVET EUROPEEN

(11) **EP 0 989 118 B1**
(45) Date de publication et mention de la délivrance du brevet: **24.10.2001**
(21) Numéro de dépôt: 99402108.7
(22) Date de dépôt: 24.08.1999
(51) Int. Cl.: C07D 201/04

(54) **Procédé de préparation de lauryllactame**
Verfahren zur Herstellung von Lauryllactam
Process for the preparation of lauryllactam

(30) Priorité: 21.09.1998 FR 9811734
(43) Date de publication de la demande: 29.03.2000
(73) Titulaire: Atofina, 92800 Puteaux (FR)
(72) Inventeur: Ollivier, Jean, 64260 Arudy (FR); Drutel, Damien, 69003 Lyon (FR)
(74) Mandataire: Neel, Henry

(56) Documents cités:
- FR-A- 1 335 822
- FR-A- 2 417 501

## Description

La présente invention se rapporte à la préparation de lauryllactame qui constitue le monomère de base du polyamide 12. Plus précisément, elle concerne un procédé de préparation de lauryllactame à partir de cyclododécane dans lequel les étapes de photonitrosation et de transposition de Beckmann sont mises en oeuvre en présence d'acide méthanesulfonique.

Le lauryllactame est largement utilisé pour la préparation du polyamide 12. Sa production à une échelle industrielle est bien connue (voir par exemple "Procédé de Pétrochimie", tome 2, pp.316-322, Edition Technip, 1986). On peut par exemple fabriquer le lauryllactame à partir de cyclododécane (procédé HÜLS et Ato Chimie), de cyclododécanone (procédé UBE) ou encore de monoozonide du cyclododécatriène (procédé Snia Viscosa).

Dans le procédé développé par Ato Chimie, on obtient le lauryllactame en deux étapes:
- dans la première étape, on forme le chlorhydrate de l'oxime de la cyclododécanone par photonitrosation du cyclododécane solubilisé dans un solvant chloré selon la réaction suivante: 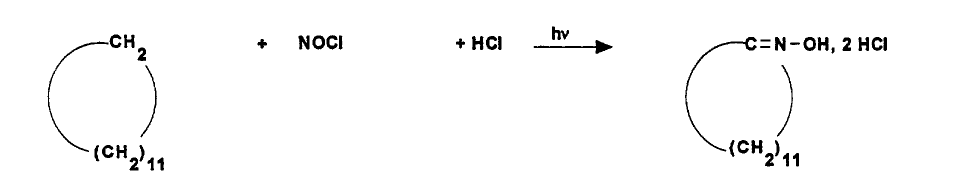
- et, dans la deuxième étape, on soumet le produit de réaction à une transposition de Beckmann en présence d'acide sulfurique:

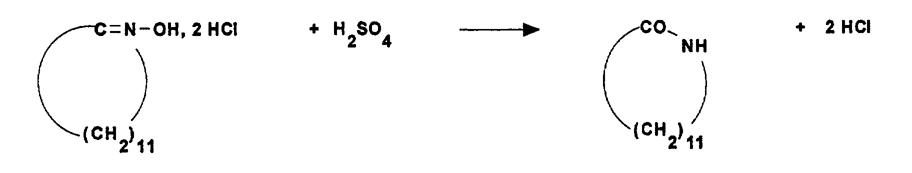

Le chlorhydrate d'oxime de la cyclododécanone formé au cours de la photonitrosation est solide et très stable, et il se dépose sur les parois des lampes d'irradiation en contact avec le milieu réactionnel. Sous l'effet de la lumière, le dépôt se transforme progressivement en une substance goudronneuse qui, à plus ou moins long terme, entraîne l'arrêt de la réaction photochimique.

Pour surmonter cette difficulté, il est connu d'ajouter de l'acide sulfurique dans le mélange réactionnel (voir par exemple FR-B-1 335 822 et FR-B- 1 553 268). De cette manière, l'acide sulfurique extrait le chlorhydrate d'oxime de la cyclododécanone au fur et à mesure de sa formation. Tout risque de dépôt étant ainsi écarté, il devient possible d'effectuer la réaction en continu.

L'emploi de l'acide sulfurique présente cependant des inconvénients, tant au niveau de la première que de la deuxième étape du procédé.

Dans l'étape de photonitrosation, l'acide sulfurique:
- colore le milieu réactionnel provoquant ainsi une perte du nombre des photons indispensables à la réaction,
- se disperse difficilement dans le milieu réactionnel du fait de sa viscosité élevée et de son caractère ionisé,
- réagit avec le chlorure de nitrosyle pour former du sulfate acide de nitrosyle, lequel sulfate contribue à dégrader le chlorhydrate de l'oxime,
- hydrolyse le chlorhydrate de l'oxime en cyclododécanone et hydroxylamine, et
- réagit avec l'agent nitrosant ce qui a pour effet de retarder le démarrage de la réaction et, par voie de conséquence de réduire la production.

Dans l'étape de transposition de Beckmann, la réaction est effectuée à une température élevée, supérieure à 135°C. De ce fait, les inconvénients liés à l'emploi d'acide sulfurique sont les suivants:
- en présence de composés organiques, l'acide sulfurique se décompose en libérant du dioxyde de soufre indésirable dont la teneur augmente au cours du recyclage de la phase organique contenant le cyclododécane n'ayant pas réagi,
- une partie de l'oxime est hydrolysée en la cétone correspondante,
- une partie du lactame est hydrolysé en l'acide aminé correspondant qui, dans les conditions du procédé, ne peut être récupéré et valorisé, et
- l'acide sulfurique est susceptible de décomposer le solvant chloré de la réaction entraîné par la phase acide en phosgène toxique pour l'homme.

De plus, tous les effluents contenant de l'acide sulfurique générés par le procédé industriel ne peuvent être recyclés qu'au prix d'un traitement long, difficile et coûteux, du fait notamment qu'il est nécessaire de mettre en oeuvre des étapes de concentration de l'acide et d'élimination des composés organiques.

Il a maintenant été trouvé que l'on peut pallier les inconvénients précités en remplaçant avantageusement l'acide sulfurique par l'acide méthanesulfonique, sans pour autant que le rendement global du procédé en soit affecté.

L'invention a donc pour objet un nouveau procédé de préparation de lauryllactame qui consiste à:
a- faire réagir photochimiquement le cyclododécane solubilisé dans un solvant organique, un agent nitrosant et du chlorure d'hydrogène en présence d'un acide pour former l'oxime de la cyclododécanone, et
b- soumettre ladite oxime à une transposition de Beckmann en présence d'un acide,
ledit procédé étant caractérisé en ce que l'acide mis en oeuvre est l'acide méthanesulfonique.

Le procédé selon l'invention sera mieux compris à la lumière de la description qui suit.

### Etape de photonitrosation

Pour effectuer la réaction, on procède en général dans un réacteur dans lequel on introduit un mélange comprenant du cyclododécane en solution dans un solvant organique et de l'acide méthanesulfonique, du chlorure d'hydrogène et un agent nitrosant, et on irradie avec de la lumière.

Le cyclododécane peut être obtenu selon des méthodes connues de l'homme du métier, par exemple par cyclotrimérisation du butadiène et hydrogénation du cyclotriène formé.

Le cyclododécane est solubilisé dans un solvant organique approprié, par exemple un hydrocarbure chloré tel que le chloroforme et le chlorobenzène. La teneur en cyclododécane dans le solvant est généralement comprise entre 0,1 et 40 % en poids et de préférence 20 et 30 % en poids.

L'acide méthanesulfonique est généralement employé sous la forme d'une solution aqueuse dont la teneur peut varier entre 70 et 90 % en poids, de préférence 95 et 99 % en poids.

L'acide méthanesulfonique mis en oeuvre représente en général 6 à 12 % du volume du milieu réactionnel, et de préférence 8 à 9 %.

L'agent nitrosant est choisi parmi le chlorure de nitrosyle, un mélange d'oxyde nitrique et de chlore, et les composés susceptibles de former du chlorure de nitrosyle dans le milieu réactionnel, par exemple des alkyl nitrites qui réagissent avec le chlorure d'hydrogène. On utilise de préférence le chlorure de nitrosyle.

On règle l'addition de l'agent nitrosant de manière que sa concentration dans le milieu réactionnel soit comprise entre 0,1 et 25 g/l et de préférence 1 et 2 g/l.

Le chlorure d'hydrogène est introduit sous la forme de gaz anhydre, en excès par rapport à l'agent nitrosant. De préférence, il est mis en oeuvre à saturation de la solution de cyclododécane dans le solvant.

L'irradiation est réalisée au moyen d'une ou plusieurs lampes à vapeur de mercure ou de sodium émettant des radiations de longueur d'onde comprise entre 500 et 700 nm, et de préférence 565 et 620 nm.

La réaction est effectuée à une température comprise entre -20 et +40°C, et de préférence +10 et +20°C.

On opère en général sous une agitation vigoureuse. Dans la présente invention, l'expression "agitation vigoureuse" s'entend d'une agitation telle que le volume réactionnel est renouvelé au moins 100 fois par heure. On peut pour cela utiliser tout moyen d'agitation, par exemple une ou plusieurs turbines ou pompes de recirculation.

La photonitrosation est généralement réalisée dans un réacteur pouvant fonctionner de manière discontinue ou continue. On préfère opérer en continu.

Après l'irradiation, le mélange réactionnel est décanté et l'on récupère l'oxime de la cyclododécanone dans la phase acide. La teneur en oxime de la cyclododécanone dans la phase acide peut varier dans une large mesure. Toutefois, pour des raisons liées aux conditions industrielles, on préfère une teneur en oxime comprise entre 10 et 40 % en poids, et mieux encore 25 et 35 %.

### Etape de transposition de Beckmann

Cette étape est généralement effectuée dans un réacteur opérant à chaud et sous une agitation vigoureuse.

La cyclododécanone oxime obtenue à l'issue de l'étape précédente de photonitrosation est généralement introduite telle quelle dans le réacteur. Pour des raisons évidentes de sécurité liées à la très forte exothermicité de la réaction, on préfère introduire la solution d'oxime dans un réacteur qui contient un volume adéquat d'acide méthanesulfonique maintenu à la température requise pour effectuer la transposition. Ce volume peut, comme le sait l'homme du métier, varier dans une large mesure selon que la réaction est mise en oeuvre de manière continue ou discontinue.

On procède en général à une température comprise entre 120 et 180°C, de préférence 140 et 160°C, et pendant une durée telle que le temps de séjour dans le réacteur varie de 2 minutes à 1 heure, de préférence 15 à 30 minutes.

La transposition est mise en oeuvre dans des conditions d'agitation vigoureuse telle que définie ci-avant.

On récupère ainsi une solution de lauryllactame dans l'acide méthanesulfonique. Cette solution est généralement soumise à un ou plusieurs traitements de séparation et de purification pour obtenir le lauryllactame ayant une pureté supérieure à 99 %.

On peut facilement purifier l'acide méthanesulfonique récupéré, par exemple par simple distillation, et le réintroduire dans le procédé.

Les exemples qui suivent permettent d'illustrer l'invention.

### EXEMPLE

### a- Photonitrosation

Dans un réacteur de deux litres (volume utile) équipé d'une lampe à vapeur de sodium ayant une puissance de 400 watts et émettant un maximum de radiations au voisinage de 595 nm, on introduit en continu une solution de cyclododécane dans le chloroforme (450 g/l ; 1 l/h), de l'acide chlorhydrique gazeux anhydre à saturation, du chlorure de nitrosyle et une solution aqueuse d'acide méthanesulfonique à 90 %. Le débit de chlorure de nitrosyle est réglé de manière à ce que la concentration dans le réacteur soit maintenue à 2 g/l de milieu réactionnel. Le volume de la solution d'acide méthanesulfonique introduit représente 10% du volume total du milieu réactionnel.

Les effluents gazeux issus du réacteur sont dirigés vers un condenseur (récupération du solvant) et un barboteur contenant une solution de soude (piégeage de l'acide chlorhydrique).

Le milieu réactionnel est soutiré en continu à raison de 1,1 l/h environ et décanté. En régime stationnaire, on récupère 0,52 mole/h de chlorhydrate d'oxime de la cyclododécanone et 0,00867 mole/h de chlorhydrate d'oxime de la chlorocyclododécanone dans la phase aqueuse, 0,0208 mole/h de monochlorocyclododécane et 8,25 x 10⁻⁴ mole/h de dichlorocyclododécane dans la phase organique.

Le nombre de moles de cyclododécane converti en heure est égal à 0,55. La sélectivité molaire en chlorhydrate d'oxime de la cyclododécanone est égale à 0,928 calculée sur la base du cyclododécane ayant réagi.

### b- Transposition de Beckmann

A 100 g d'acide méthanesulfonique maintenu à 120°C et sous agitation, on ajoute en 1 heure 231 g de la phase acide décantée de l'étape a- qui contient 31 % en poids d'oxime de la cyclododécanone (0,363 mole). Le milieu réactionnel est porté à 135-140°C pendant 1 heure pour parachever la transposition.

Au milieu réactionnel, on ajoute de l'eau (30 % en poids) pour faire précipiter le lauryllactame et on filtre. Le gâteau de filtration est dissous dans un mélange cyclohexane/toluène (50/50 v/v) et recristallisé. L'opération est renouvelée deux fois.

On récupère ainsi 70,9 g de lauryllactame (rendement: 99 %).

Le rendement total de la synthèse du lauryllactame est égal à 91,8 % (0,928 x 99 %).

### EXEMPLE COMPARATIF

On procède dans les conditions de l'exemple précédent modifiées en ce que l'on remplace l'acide méthanesulfonique par de l'acide sulfurique.

### a- Photonitrosation

En régime stationnaire, on récupère 0,433 mole/h de chlorhydrate d'oxime de la cyclododécanone et 0,011 mole/h de chlorhydrate d'oxime de la chlorocyclododécanone dans la phase aqueuse, 0,016 mole/h de monochlorocyclododécane et 5 x 10⁻⁴ mole/h de dichlorocyclododécane dans la phase organique. Le nombre de moles de cyclododécane converti en une heure est égal à 0,495.

La sélectivité molaire en chlorhydrate d'oxime de la cyclododécanone est égale à 0,875 calculé sur la base du cyclododécane ayant réagi.

### b- Transposition de Beckmann

A 100 g d'acide sulfurique à 98 % maintenu à 120°C et sous agitation, on ajoute en une heure 250 g de la phase acide décantée de l'étape a- qui contient 30 % en poids d'oxime de la cyclododécanone (0,38 mole). Après 1 heure à 135-140°C, le milieu réactionnel contient outre le lauryllactame, 1,125 g de cyclododécanone, 0,75 g d'acide 12-aminododécanoïque et environ 500 ppm de dioxyde de soufre. Les gaz de transposition contiennent du dioxyde de soufre (pour une quantité correspondant à la décomposition d'environ 1 % de l'acide sulfurique de départ) et plusieurs dizaines de ppm de phosgène.

Après extraction dans les conditions de l'exemple 1, on récupère 73,12 g de lauryllactame (rendement : 97,5 %).

Le rendement total de la synthèse de lauryllactame est égal à 85 %.

## Revendications

1. Procédé de préparation de lauryllactame qui consiste à:
a- faire réagir photochimiquement le cyclododécane solubilisé dans un solvant organique, un agent nitrosant et du chlorure d'hydrogène en présence d'acide pour former l'oxime de la cyclododécanone,
b- soumettre ladite oxime à une transposition de Beckmann en présence d'acide,
ledit procédé étant **caractérisé en ce que** l'acide est l'acide méthanesulfonique.

2. Procédé selon la revendication 1, **caractérisé en ce que** le solvant organique est un hydrocarbure chloré.

3. Procédé selon la revendication 2, **caractérisé en ce que** le solvant est le chloroforme ou le chlorobenzène.

4. Procédé selon l'une des revendications 1 à 3, **caractérisé en ce que** l'acide méthanesulfonique dans l'étape a- représente 6 à 12 % du volume du milieu réactionnel.

5. Procédé selon la revendication 4, **caractérisé en ce que** l'acide méthane sulfonique représente 8 à 9 % du volume réactionnel.

6. Procédé selon l'une des revendications 1 à 5, **caractérisé en ce que** l'oxime de l'étape b- est sous la forme d'une solution contenant 10 à 40 % en poids d'oxime dans l'acide méthanesulfonique.

7. Procédé selon la revendication 6, **caractérisé en ce que** la solution contient 25 à 35 % en poids d'oxime.

## Patentansprüche

1. Verfahren zur Herstellung von Lauryllactam, das in folgenden Stufen besteht:
a- photochemische Umsetzung von in einem organischen Lösungsmittel gelöstem Cyclododecan, einem Nitrosierungsmittel und Chlorwasserstoff in Gegenwart von Säure unter Bildung des Oxims des Cyclododecanons
und
b- Beckmann-Umlagerung des Oxims in Gegenwart von Säure, wobei das Verfahren **dadurch gekennzeichnet ist, daß** die Säure Methansulfonsäure ist.

2. Verfahren nach Anspruch 1, **dadurch gekennzeichnet, daß** das organische Lösungsmittel ein chlorierter Kohlenwasserstoff ist.

3. Verfahren nach Anspruch 2, **dadurch gekennzeichnet, daß** das Lösungsmittel Chloroform oder Chlorbenzol ist.

4. Verfahren nach einem der Ansprüche 1 bis 3, **dadurch gekennzeichnet, daß** die Methansulfonsäure in Stufe a- 6 bis 12 % des Volumens des Reaktionsmediums ausmacht.

5. Verfahren nach Anspruch 4, **dadurch gekennzeichnet, daß** die Methansulfonsäure 8 bis 9 % des Reaktionsvolumens ausmacht.

6. Verfahren nach einem der Ansprüche 1 bis 5, **dadurch gekennzeichnet, daß** das Oxim von Stufe b- in Form einer Lösung vorliegt, die 10 bis 40 Gew.-% Oxim in der Methansulfonsäure enthält.

7. Verfahren nach Anspruch 6, **dadurch gekennzeichnet, daß** die Lösung 25 bis 35 Gew.-% Oxim enthält.

## Claims

1. Process for the preparation of lauryllactam which consists in:
a- photochemically reacting cyclododecane solubilized in an organic solvent, a nitrosing agent and hydrogen chloride in the presence of acid in order to form cyclododecanone oxime,
b- subjecting the said oxime to a Beckmann rearrangement in the presence of acid,
the said process being **characterized in that** the acid is methanesulphonic acid.

2. Process according to Claim 1, **characterized in that** the organic solvent is a chlorinated hydrocarbon.

3. Process according to Claim 2, **characterized in that** the solvent is chloroform or chlorobenzene.

4. Process according to one of Claims 1 to 3, **characterized in that** the methanesulphonic acid in stage a- represents 6 to 12% of the volume of the reaction medium.

5. Process according to Claim 4, **characterized in that** the methanesulphonic acid represents 8 to 9% of the reaction volume.

6. Process according to one of Claims 1 to 5, **characterized in that** the oxime of stage b- is in the form of a solution containing 10 to 40% by weight of oxime in methanesulphonic acid.

7. Process according to Claim 6, **characterized in that** the solution contains 25 to 35% by weight of oxime.
